# EUROPEAN PATENT APPLICATION

(11) **EP 1 372 102 A2**
(43) Date of publication of application: **17.12.2003**
(21) Application number: 03253709.4
(22) Date of filing: 12.06.2003
(51) Int. Cl.: G06F 19/00

(54) **Interactive patient data report generation**

(30) Priority: 13.06.2002 US 172420
(71) Applicant: Lifescan, Inc., Milpitas, California 95035-6312 (US)
(72) Inventor: Matian, Gregor, Foster City, California 94404 (US); Kugizaki, Rodney James, San Ramon, California 94583 (US)
(74) Representative: Mercer, Christopher Paul

(57) **Abstract**

Systems and methods for patient data report generation and patient data management that utilize interactive reports for patient data associated with health disorders such as diabetes, blood clotting disorders, and the like. The invention also provides systems and methods of monitoring health disorders using networked computer technology. The methods comprise selecting a report presentation format, displaying patient data in the report presentation format, selecting a data feature associated with the patient data, and displaying the selected data feature, and may additional comprise editing or revising the selected data feature. Selected data features may be displayed by visual expansion, by pop-windows, by separate hyperlinked display pages, or in other display format.

## Description

### INTRODUCTION

### Background of the Invention

Software applications that display medical or patient information in a database or repository have traditionally used the concept of "canned reporting". A typical report generation session involves user selection of one or more pre-formatted reports from a list of available reports, to which a user provides criteria to control the content of the report. The patient information is then compiled and placed in the pre-determined format of the selected report and displayed for the user. If the report in its final form does not include or does not properly show the particular information of interest to the user, the entire process must be repeated and a new report generated.

This process is rigid and inflexible, and often requires several iterations in order to make relatively minor changes in the final report. Often, the user does not know what problem needs to be solved until the final report has been generated. If the report does not meet the user's needs, a different report, a complete new report must be generated. Since the user does not know in advance of report generation what issues will be revealed in a report several passes or iterations of report generation may be required with each such pass requiring the user to repeat the entire report generation process, may be required to ultimately generate a report that optimally presents the data of interest.

Thus there is a need for patient data report generating systems and methods that simplifies data interpretation, that allows for the selection of intuitive report parameters, which eliminates the need to generate multiple additional reports in order to obtain a desired report feature, and which allows quick and easy sharing of patient data information between patients, consented caregivers, consented healthcare providers and other consented users. The present invention satisfies these needs, as well as others, and overcomes the deficiencies found in the background art.

### Relevant Literature

U.S. Patent documents of interest include 6,024,699, 5,960,403, 5,997,476 and 5,935,060.

### SUMMARY

The invention provides systems and methods for patient data report generation and patient data management that utilize interactive reports for patient data associated with health disorders such as diabetes, blood clotting disorders, and the like. The invention also provides systems and methods of monitoring health disorders using networked computer technology. The subject methods comprise, in general terms, selecting a report presentation format, displaying patient data in the report presentation format, selecting a data feature associated with the patient data, and displaying the selected data feature. The methods may further comprise editing or revising the selected data feature. The displaying of the selected data feature may comprise expanding the selected data feature, contracting the selected data feature, or other visual manipulation of the selected data feature. Displaying the patient data in the report presentation format may comprise generating a first visual display page, while displaying the selected data feature comprises displaying a second visual display page hyperlinked to the first visual display page. Displaying of the selected data feature may be carried out via one or more drill-down operations. In other embodiments, displaying the selected data feature may comprise displaying the selected data feature in a window overlaying the patient data report presentation format. The methods may comprise generating patient data for use in the selected report formats. Generating patient data may comprise uploading patient data from a sample reader or downloading patient data from a file server, or transmitting, obtaining or receiving patient data as an email attachment or as a direct facsimile transmission of the patient data in electronic form.

The systems of the invention comprise, in general terms, programming configured to allow a user to select a data report presentation format, programming configured to display patient data in the selected data report presentation format via a visual interface, programming configured to allow the user to select a data feature associated with the patient data selected data feature via the visual interface. The systems may further comprise programming configured to allow the user to edit the selected data feature. The programming configured to display the selected data feature may comprise programming configured to expand, contract, or otherwise manipulate the selected data feature in a desired manner. In certain embodiments, the programming configured to display patient data, the programming configured to allow the user to select a data feature, and the programming configured to display the selected data feature may be configured to display the patient data in the report presentation format as a first visual display page, and to display the selected data feature as a second visual display page hyperlinked to the first visual display page. Such display generation may be in the form of a drill down operation. In other embodiments, the programming configured to display the selected data feature may be configured to visually present the selected data feature to a user in a window overlaying the patient data report presentation format.

### BRIEF DESCRIPTION OF THE DRAWINGS

The invention will be more fully understood by reference to the following drawings, which are for illustrative purposes only.

Figure 1 is a functional block diagram illustrating one embodiment of an interactive patient data management system in accordance with the invention.

Figure 2 is a flow chart illustrating an interactive patient data report generation method in accordance with the invention.

Figure 3A and FIG. 3B are illustrations of the visual display for a patient data report with tabular and graphical pie chart representations of patient blood glucose data.

Figure 4A and FIG. 4B are illustrations of the visual display for a patient data report with a standard day chart representation.

Figure 5A and FIG. 5B are illustrations of the visual display for a patient data report with a trend chart representation.

FIG. 6 is a flow chart illustrating another embodiment of an interactive patient data report generation method in accordance with the invention.

FIG. 7 is an illustration of a visual page display of a patient data report in a data list format.

FIG. 8 is an illustration of a visual page display of a day view of a selected day from the page display of FIG. 7.

FIG. 9 is an illustration of a display page of a selected data entry from the page display of FIG. 8.

### DEFINITIONS

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It should be noted that as used herein and in the appended claims, the singular forms "a", "and", and "the" include plural referents unless the context clearly dictates otherwise. Thus, for example, reference to "a user" includes a plurality of such users and reference to "the server" includes reference to one or more and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Any definitions herein are provided for reason of clarity, and should not be considered as limiting. The technical and scientific terms used herein are intended to have the same meaning as commonly understood by one of ordinary skill in the art to which the invention pertains.

As used herein, "user" and grammatical equivalents thereof refers to any individual, person, or entity wishes to generate a report based on patient data Non-patient users of patient data may be subject to permission and/or the consent from patients. A "user" may comprise a patient, patient relative, caregiver, health professional, or any other person desiring to create patient data reports and view patient data.

### DETAILED DESCRIPTION OF THE INVENTION

Disclosed herein are systems and methods for interactive patient management that permit patients, caregivers, healthcare providers or other users to quickly and easily analyze and interpret a patient's data. The invention allows patient data to be presented, via visual display, in a variety of selectable report formats. The patient data reports are interactive and user-friendly, and permit users to select, expand and edit individual data features present in a report format in a simple "point-and-click" manner. Patient data reports and selected data features therein can thus be quickly and easily revised or edited, and visually reviewed during such revisions, to provide effective report writing and patient data management.

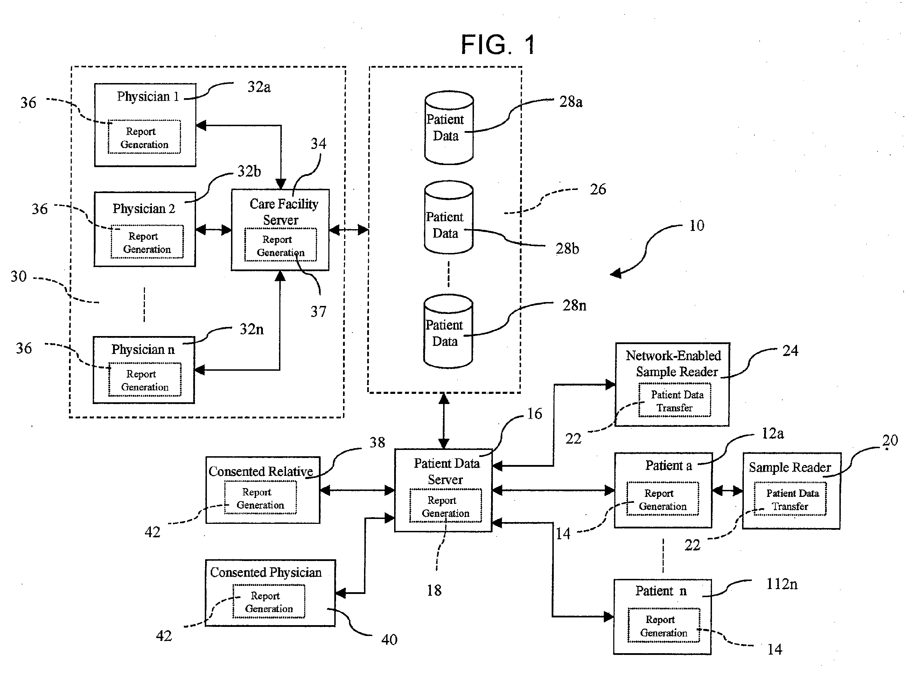

Reference is made more specifically to the drawings in which, for illustrative purposes, show the present invention embodied in systems and methods in FIG. 1 through FIG. 5. It will be appreciated that the systems may vary as to configuration and as to details of the parts, and that the methods may vary as to detail and the order of the events or acts, without departing from the basic concepts as disclosed herein. The invention is disclosed primarily in terms of use with patient data information, and particularly in terms of use for management of patients with diabetes and anti-coagulate health conditions. The invention may be used, however, in the management of any form of data and for report generation for such data, including data associated various health conditions in which routine monitoring of the condition or an analyte is needed, such as high blood pressure and associated exercise regimes for obesity. The invention is also described in terms of users being patients, physicians and other health professionals, and it should be understood that the users of the invention need not necessarily be the patient or a health professional, but may be any user which has the consent of the patient or patient guardian to access the patients health data, such as patient relatives, caregivers, medical researchers and the like. It should also be apparent to those skilled in the art that various functional components of the invention as described herein-may share the same logic and be implemented within the same circuit, or in different circuit configurations. Any definitions herein are provided for reason of clarity, and should not be considered as limiting, and any technical and scientific terms used herein are intended to have the same meaning as commonly understood by those skilled in the art.

The software aspects of the invention may be distributed in nature, and may be embodied in many configurations within client-server computer systems. The invention may be embodied in a variety of stand-alone as well as networked computer or data processor systems. In some embodiments, the subject patient data management methods may be carried out in association with a stand alone program operating on a single computer or data processing device, while in other embodiments a portion or all of the patient management methods may be carried out in association with a web-based application residing on one or more server computers that are accessed by a users computer via network interface.

### Systems

Referring now to FIG. 1, there is shown one embodiment of a patient data management system 10 in accordance with the invention that provides interactive patient data report generation. The system 10 includes one or more patient computers 12a, 12n, each of which may comprise a standard computer such as a minicomputer, a microcomputer, a UNIX® machine, mainframe machine, personal computer (PC) such as INTEL® , APPLE® , or SUN® based processing computer or clone thereof, or other appropriate computer. Patient computers 12a, 12n may also include typical computer components (not shown), such as a motherboard, central processing unit (CPU), memory in the form of random access memory (RAM), hard disk drive, display adapter, other storage media such as diskette drive, CD-ROM, flash-ROM, tape drive, PCMCIA cards and/or other removable media, a monitor, keyboard, mouse and/or other user interface, a modem, network interface card (NIC), and/or other conventional input/output devices. In many embodiments, patient computers 12a, 12n comprise conventional desktop or "tower" machines, but in certain embodiments can alternatively comprise portable or "laptop" computers, handheld personal digital assistants (PDAs), cellular phones capable of browsing Web pages, "dumb terminals" capable of browsing Web pages, internet terminals capable of browsing Web pages such as WEBTV® , or other Web browsing or network enabled devices.

Each patient computer 12a, 12n may comprise, loaded in its memory, an operating system (not shown) such as UNIX® , WINDOWS® 98, WINDOWS® ME, WINDOWS® 2000 or the like. Each patient computer 12a, 12n may further have loaded in memory a Web Browser program (not shown) such as NETSCAPE NAVIGATOR® , INTERNET EXPLORER® , AOL® , or like browsing software for patient computers. In accordance with the invention, patient computers 12a, 12n may each comprise a data report generating software element, application or programming 14 stored in memory that allows users of patient computers 12a, 12n to selectively input patient data into a logbook format for a specific health condition such as diabetes. Programming 14 provides for interactive patient data report generation, and is configured to allow users to select various data presentation formats, compile patient data in the selected presentation format, display data in the selected format, and select, expand on and/or revise or modify selected data features prior to generating a finalized report as described further below. Programming 14 may be the form of electronically, optically, or magnetically stored code or other form of computer readable stored code, that is loaded in the RAM or other memory of patient computers 12a-12n. In the embodiment shown in FIG. 1, each patient computer 12a-12n represents a computer used by an individual for the inputting of patient data as well as for interactive reporting and data interpretation.

The system 10 also comprises one or more patient data servers or file servers 16, which may be any standard data processing device or computer, including a minicomputer, a microcomputer, a UNIX® machine, a mainframe machine, a personal computer (PC) such as INTEL® based processing computer or clone thereof, an APPLE® computer or clone thereof or, a SUN® workstation, or other appropriate computer. While only one patient data server 16 is shown, many such patient data servers 16 may be present in the system 10 as required by the number of users and traffic levels. Patient data server 16 may include conventional computer components (not shown) such as a motherboard, central processing unit (CPU), random access memory (RAM), hard disk drive, display adapter, other storage media such as diskette drive, CD-ROM, flash-ROM, tape drive, PCMCIA cards and/or other removable media, a monitor, keyboard, mouse and/or other user interface means, a modem, network interface card (NIC), and/or other conventional input/output devices.

Patient data server 16 has stored in its memory a server operating system (not shown) such as UNIX® , WINDOWS® NT, NOVELL® , SOLARIS® , or other server operating system. Patient data server 16 also has loaded in its memory web server software (also not shown) such as NETSCAPE® , INTERNET INFORMATION SERVER™ (IIS), or other appropriate web server software loaded for handling HTTP (hypertext transfer protocol) or Web page requests. Patient data server 16 may also comprise a stored data report generation software element, application or programming 18 for creation of patient data reports in accordance with the invention. Programming 18 may include some or all of the aspects of programming 14, and may provide such programming aspects to users of patient computers 12a-12n embedded within HTML pages. Programming 18 may be in the form of electronically, optically, or magnetically stored code or other form of computer readable stored code, that is loaded in the RAM or other memory of patient data server 16. Programming 18 may also be an element of patient computer 12a and 12b in certain embodiments.

Patient computers 12a and 12n are operatively coupled to patient data server 16 for communication with patient data server 16 via the Internet, Intranet, LAN, WAN (not shown) or other computer network using DSL (digital subscriber line), telephone connection with a modem and telephone line via an internet service provider (ISP), wireless connection, satellite connection, infrared connection, or other means for establishing a connection to the Internet. Patient data server 16 may be connected to the Internet by a fast data connection such as T1, T3, multiple T1, multiple T3, or other data connection. Patient computers 12a, 12n and patient data server 16 communicate via the Internet or other network connection using the TCP/IP (transfer control protocol/internet protocol) or other network communication protocol.

The system 10 may include one or more sample readers 20 used in association with patient computers 12a-12n. Sample reader 20 is capable of measuring and recording patient data, and thus may include an interface (not shown) that accepts patient samples, which may be in the form of bodily fluid samples such as blood, urine, sputum and the like, as well as hardware and/or software (also not shown) that enables sample reader 20 to record and store patient data associated with the samples. Sample reader 20 comprises programming 22 that allows storage of patient data obtained by sample reader 20, as well as provide for interactive patient data report generation by allowing users to select various data presentation formats, display data in the selected format, and select, expand on and/or edit the selected data features. Programming 22 may be capable of presenting these operations to a user via a visual interface on sample reader 20, and/or a handheld device capable of rendering the data provided by the sample reader, and/or on the visual interface of a computer 12a operatively coupled to sample reader 20. Programming 22 may additionally, or alternatively, be configured to transfer patient data to patient computer 12a-12n for use by report generation programming 14 of patient computer 12a, by report generation programming 18, or other report generation programming as described further below.

System 10 is also shown as including a network-enabled sampler reader or meter 24 that is capable of measuring and recording patient data. Sample reader 24 includes an interface (not shown) that accepts patient samples and hardware and/or software (also not shown) that enables sample reader 20 to record and store patient data associated with the samples in the same manner as described for sample reader 20 above. Network-enabled sample reader 24 is operatively coupled to patient data server 16 for communication with patient data server 16 via network connection, which may comprise a telephone connection with a modem and telephone line to directly dial up patient data server 16, a wireless connection, satellite connection, infrared connection, or other means for establishing a connection to a server. Network-enabled sample reader 24 comprises programming 22 configured to allow users to select various data presentation formats, display data in the selected format, and select, expand on and/or edit the selected data features. These operations may be presented to a user via a visual interface on sample reader 24 and/or on the visual interface of another computer operatively coupled to sample reader 20 as described above. Programming 22 may additionally, or alternatively, be configured to transfer patient data stored in reader 24 to server 16 for use by programming 18 for patient data report generation. Programming 22 may include browser capability, and may additionally be capable of receiving or accepting a completed report from server 16 and displaying the report on a display interface (not shown) of reader 24. Network enabled sample reader 24 allows patients that do not have access to a personal computer to provide patient data to server 16 for report generation via programming 18 on server 16.

The system 10 includes a data bank 26 that may comprise one or more individual databases 28a, 28b, 28n which are operatively coupled to patient data server 16. Patient data server 16, in this regard, may include stored database management programming such as SQL® , DB2® or like programming capable of retrieving and storing information in association with databases 28a, 28b, 28n. Patient data server 16 alternatively may be operatively coupled to databases 28a, 28b, 28n through one or more database servers (not shown) that are capable of accessing information from databases 28a, 28b, 28n.

Databases 28a-28n may include any stored data usable in the generation of patient data reports. Such data may comprise, inter alia, patient data in the form of periodic measurements made during the monitoring of patient medical conditions. For example, the data may comprise periodic levels of analyte found in a patient bodily fluid. The analyte may be indicative of a disease condition or of the level or amount of a therapeutic compound present in the bodily fluid. In specific instances, the patient data may comprise blood glucose levels that are periodically determined for diabetic patients or anticoagulant levels measured in the blood or serum of patients undergoing anticoagulation therapies. This data may include information associated with the timing or other events associated with measurements, such as the timing of patient meals with respect to the taking of measurements, the time of day or night of measurements, patient exercise regimens, menstrual cycle, other medical conditions of patients or other drug therapies to which a patient may subscribe, or various other factors or considerations that may be used in patient data report generation and the selection of specific data features in association with report generation.

Stored information in databases 28a, 28b, 28n may specifically comprise information usable for selection of patient data report parameters, such as the number of data readings to be taken per day, average statistical readings of such data, the number of days of data used to produce a report, and other report parameters relating specifically to the health condition being monitored and the management thereof. Stored information for report parameters for managing a specific disease such as diabetes may comprise, for example, established blood glucose values associated with various types of insulin treatments, blood glucose levels associated with age of the patient, and identification of dangerous blood glucose levels. Non-patient users which have patient consent to access the patient data information in databases 28a, 28b, 28n may use the patient data to create reports on a group of individuals for research and other medical purposes.

Data bases 28a, 28b, 28n are also configured to store previously selected report parameters from repeat users for subsequent use by the same users or others for preparing completed patient reports. Databases 28a-28n may comprise, in whole or in part, proprietary databases comprising raw data from monitoring devices for a plurality of patients as well as compiled results and reports for individuals or groups of individuals with related health conditions. The databases 28a-28n may be created directly by patients who may periodically update databases 28a-28n via upload from sample readers 20, 24 or patient computer 12a-12n, or by health professionals or caregivers for use by patients and others (subject to patient consent) to access health data.

The stored information in databases 28a, 28b, 28n may also comprise information related to selection of data points parameters, including the number of data points selected, time intervals between data point readings, standard values for control or averaged data points, or other control considerations for comparison studies. Databases 28a, 28b, 28n may additionally comprise information usable for selection of the layout of report parameters such as general layout patterns for various health conditions, the number of interactive features per report, various visual representations of report results and other report layout considerations.

The information in databases 28a, 28b, 28n may be configured in a variety of arrangements known to those skilled in the art. The databases 28a, 28b, 28n may comprise relational databases wherein report parameter selection information, control or standard reading parameter selection information, and data point parameter selection information are arranged as tables of records stored in computer-readable media.

The system 10 as shown in FIG. 1 includes a care facility local area network or LAN 30 that comprises one or more physician computers 32a, 32b, 32n operatively coupled to a server 34 within LAN 30. Physician computers 32a-32n may comprise any of the computer or data process devices described above for patient computers 12a-12n, with conventional operating system and browser software as described above, and care facility LAN server 34 may comprise a computer configured in a manner similar to patient data server 16 described above. Care facility LAN server 34 is operatively coupled to databases 28a, 28b, 28n via the Internet or other computer network. A firewall (not shown) may be used in association with LAN 30 for filtering inbound and outbound traffic. Care facility LAN server 34 may include web server software (not shown) for handling HTTP page requests from Physician computers 32a-32n, as well as software (also not shown) for storing and retrieving information in association with databases 28a-28n.

Physician computers 32a, 32b, 32n may each include a stored software element, application or programming 36that is configured to allow users of physician computers 32a-32n to construct and view patient data reports using selectable report formats and selection, expansion and/or editing of selectable data features, to aid in the management of the patient treatment or patient health. Programming 36 may include some or all of the logic aspects of programming 14, and allows physician users to select various data presentation formats, compile patient data in the selected presentation format, display data in the selected format, and expand on and revise or modify selected data features prior to finalizing a report. Care facility LAN server 34 may include report generation programming 37 that provides a web-based approach to interactive patient data report generation for physician computers 32a-32n, and which may be used alternatively to, or in addition to, programming 36.

The system 10 as shown in FIG. 1 has one or more computers outside of the care facility LAN 30 that are available to users that have been granted consent from the patient to access patients' data A consented patient relative computer 38, and a consented physician computer 40 are shown in this regard as operatively coupled to server 16 via the Internet or other computer network. Relative and physician computers 38 and 40 comprise any of the computer or data process devices described above with conventional operating system and browser software as described above.

The relative and physician computers 38 and 40 may also include a stored software element, application or programming 42 that is configured to allow users of the relative and physician computer 38 and 40 to create and view patient data reports using selectable report formats, and selection, expansion and/or editing of selectable data features in accordance with the invention. Programming 42 may include some or all of the logic aspects of programming 14, and allows users of computers 38, 40 to select various data presentation formats, compile patient data in the selected presentation format, display data in the selected format, and expand on and revise or modify selected data features prior to finalizing a report.

The patient data management system 10 of FIG. 1 is only one embodiment of the interactive patient data management system of the invention, and numerous variations on the system 10 will suggest themselves to those skilled in the art upon review of this disclosure. In some embodiments, the system of the invention may comprise a single computer with stored patient data and stored programming capable of carrying out the interactive report generating methods of the invention. In some embodiments, the system of the invention may reside in a single patient computer 12a and a single physician computer 40 operatively coupled through server 16. The physician user would utilize the interactive patient data report generation programming 42 and/or programming 18 to select report parameters and create reports of various kinds which aid in the managing of the patient's health condition.

The arrangement of care facility LAN 30 as shown in FIG. 1 is also only exemplary and may be varied. A separate physician computer 32a-32n is shown for comprising a separate report parameter programming 36a-36n to generate patient reports. For reason of clarity, databases 28a-28n are shown in FIG. 1 as being within a single databank 20 that is accessible by computers 32a, 32b and 32n of the care facility LAN 30 and computers 12a, 12n, 38 and 40. This arrangement of databases 28a-28n may vary in different embodiments of the invention. Physician computers 40 may be located within a separate care facility or institutional LANs (not shown). Server 16 may comprise a bank or farm of servers that is expandable and re-configurable according to the number of client users and traffic levels that arise, and various load balancing mechanisms may be used to assign particular servers to particular client users. Various other arrangements of the computers and databases within the system 10 will suggest themselves to those skilled in the art, and are considered to be within the scope of this disclosure.

Databases 28a-28n may be care facility proprietary databases wherein patient access to the databases is to be made on a subscription or fee basis. Thus, the users of patient machines 12a and 12n, and relative and physician machines 38 and 40, may pay a monthly or annual subscription fee, or pay fees on a per-report basis, for use of databases 28a-28n to access patient data information. Access to databases 28a-28n by users via patient data server 16 may be secure and subject to authorization or authentication of users prior to access. Numerous other database arrangements for the system 10 will suggest themselves to those skilled in the art, and are considered to be within the scope of this disclosure.

Input data programming 14 and/or report parameter selection programming 18 may be provided by a care facility or institution to patients on computer readable media such as a CD for installation on patient computers 12a and 12n. Alternatively, input data programming 14 and/or report parameter selection programming 18 may be downloaded to patient computers 12a and 12n from patient data server 16 or other server (not shown) via the Internet. Input data programming 14 and/or report parameter selection programming 18 may be made available to patients, physicians, caregivers or relatives on a cost basis on a subscription basis or one-time fee basis, and may be periodically upgraded by the care facility or institution according to advances in interactive report selection technology. Internet access to programming 18 on patient data server 16 may be subscription-based and subject to user authentication prior to access. Web-based programming 46 may also comprise extension application associated with third party servers (not shown) that provides access to programming 18 on patient data server 16 via the third party servers.

### Methodology

The invention provides methods that allows patients or other users to participate in the report process of patient health data as well as patient health management. The invention allows quick, easy interactive revision of various selectable aspects of the report prior to creating a final report, that allows creation of patient data reports in a desired manner without unnecessary generation of multiple reports in order to finalize a desired report. The methods of the invention, in general terms, comprise selecting a data presentation format for a report, displaying the data, expanding a selected data feature, displaying the selected data feature, and generating a completed report in the selected data presentation format. The methods may also comprise revising the selected data feature and/or revising the data presentation format, and may additionally comprise generating patient data for use in the report The methods of the invention permit patients, caregivers or other users to participate in the patient health management process to the extent desired or required by individual users. Patients may themselves provide most or substantially all of the data necessary to complete an interactive patient data report for the management of specific health conditions or other uses.

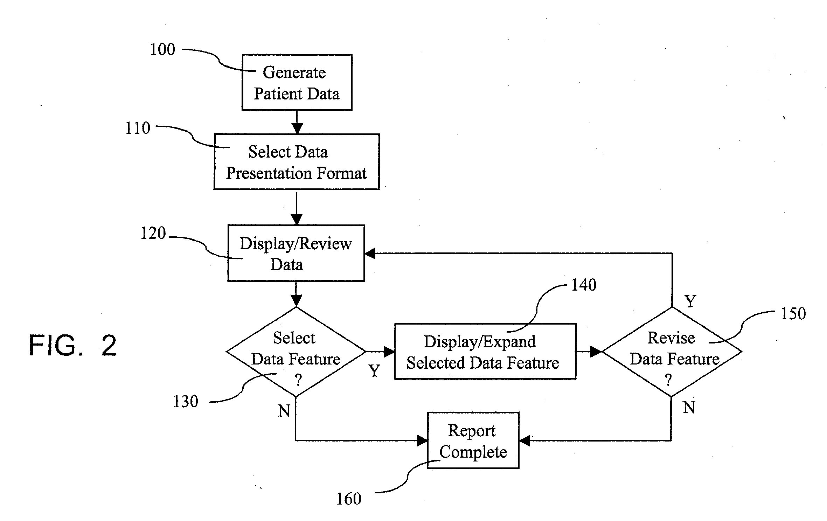

The methods of the invention will be more fully understood by reference to FIG. 2 wherein a flow chart illustrating one embodiment of the subject methods is shown. The events shown in FIG. 2 are described in terms of use with the system of FIG. 1, and particularly in terms of use by a patient user of a single patient computer 12a-12n using programming 14 associated with patient computer 12a-12n, or web-based programming 18. The report generation programming 18 associated with server 16 provides a web-based application to offer the same program or logic features present in the report generation programming 14 on client computer 12a-12n. It should be understood that users of computers 32a-n within LAN 30 may carry out the same events shown in FIG. 2 using programming 36 and/or programming 37 on server 34, which provide many or all of the same programming operations of programming 14 and 18. Similarly, users of computers 38, 40 may practice the method of FIG. 2 using programming 42 and/or programming 18.

At event 100, patient data is generated from which patient data reports may subsequently be created. The patient data may include information resulting from periodic tests of bodily fluid for detectable analytes that are indicative of a disease condition or the level of a therapeutic. Patient data may be manually entered via keyboard (not shown) associated with computer 12a-12n by a patient, or may be recorded by sample reader 20 and transferred to the memory of computer 12a. Patient data may be transmitted to and from various computers within the system 10, and imported into programming 14 and or 18 in a conventional manner. This patient data may be stored within the memory of computer 12a-12n and/or transferred to databases 28a-28n via server 16. Patient data also may be recorded by network enabled sample reader 24 and transferred to databases 28a-28n via server 16. The patient data may comprise, for example, blood glucose level information, blood serum anticoagulant level, blood or serum levels of other therapeutics, blood pressure information, or other patient data Depending upon the type of report to be generated, the patient data may be derived from a single patient or multiple patients.

At event 110, programming 14 on patient computer 12a or 12n is executed, or browser programming on patient computers 12a-12n is executed together with web-based programming 18 on patient server 16, to provide for selection of a data presentation format for patient data. The data presentation format may be in the form of a logbook or data list format of the type commonly used for diabetes patients, as described more specifically below. Other types of data presentation, including tabular or spread sheet formats, graphical presentations, trend charts, "standard day" charts, pie charts, bar graphs, and the like, may also be selected in this event. Patient and physician users may, for example, wish to select data presentation formats that reflect trends associated with an individual patient in order to patient progress or the effectiveness of treatment regimes. Medical research users may select data presentation formats usable for data from multiple patients

User selection of a particular data presentation format may be prompted by a suitable visual interface presented on the display of computer 12a-12n. The displayed visual interface may, for example, utilize "pull-down" menus to provide patient data format selections prompts to the user, "help" menus for providing instructions, graphical user interface (GUI) icons upon which a user may "click" with a mouse to make a selection, text fields in which a user may enter alphanumeric character strings using a keyboard, or other conventional visual interface tools. Where web-based programming 18 is used to create a visual user interface on the display of patient computers 12a-12n, the GUI and other visual interface tools may be based on Java applets embedded in HTML (Hypertext Markup Language) pages of programming 18 that are executed by browser programming stored on patient computers 12a-12n.

At event 120, the patient data is displayed for the user, via visual interface by programming 14 and/or 18, in the selected presentation format. This display provides the user with a view of how the data report will ultimately appear and allows the user to make additional selections regarding individual data features associated with the selected report format as described below.

At event 130 the user of computer 12a-12n may select a particular data feature or parameter of the data presentation format displayed in event 120. A selectable data feature may comprise, for example, a particular row(s) or column(s) of a tabular data presentation format, a particular cell(s) within a row or column, a selected "wedge" of a pie chart, a selected region of a graphical data representation, data associated with measurements taken on a particular day or at a particular time of day, measurements associated with resting or stimulated conditions, measurements associated with meals, or any feature, component or aspect of the patient data. If the user selects a data feature, event 140 occurs. If the user does not wish to select any data features associated with the data presented in the selected format, generation of the patient data report is complete, and event 160 may occur. Data features may be presented to the user by programming 14 and/or 18 in the form of graphical user interface (GUI) devices, "clickable" icons, manipulable first class objects, or in other conventional manner. Selection of data features in many embodiments may be carried out by "clicking" on the displayed data feature of interest or an icon representing that data feature using a "mouse".

At event 140, the data feature selected in event 130 is displayed via visual interface generated by programming 14 and/or 18. The display of the selected data feature may comprise a visually expanded version of the data feature. Such visual expansion may comprise a visual blow-up of the selected data feature. The expanded data feature-may, in some embodiments, be displayed in a separate window or frame such as a "pop-up" window, which may overlay or be superimposed on the display of the data presentation format. The expansion and display of individual data features in event 140 provides the user with the opportunity to analyze specific data and, if desired, change specific features of the data presentation format to optimize the final data report.

The selected data feature may, in certain embodiments, be presented in a separate display page. Data feature selection may involve navigation between hyperlinked pages such as HTML pages, such that the overall data report format is associated with a first display page, and selectable data features are associated with other display pages hyperlinked together. "Hyperlinked" and "hyperlinking" as used herein refers to the conventional manner of linking a program object in one computer-generated display or electronic document to another computer generated display or electronic document by "clicking" on a hyperlink icon.

One or more display pages associated with a selected data feature may be accessed in a drill-down manner. The term "drill-down" means to move from the display of summary or less detailed information, towards display of more detailed information by focusing on or selecting a specific data feature. To drill down through a series of display pages or folders, for example, means to go through a hierarchy of pages or folders to find or focus on a specific selected data feature or aspect thereof. The display of a selected data feature thus may comprise a drill-down operation or a series of drill-down operations to ultimately provide the user with a data feature of interest. Drill -down operations may involve subsequent selection of data features that are within or a subpart of a previously selected data feature, as described further below.

In certain embodiments, visual display of the selected data feature may alternatively comprise a contraction or reduction of the data feature such that the displayed feature is reduced in size.

At event 150, the user may elect to revise or otherwise change the selected data feature displayed in event 140. Revision of the data feature in event 150 may be carried out by user by, for example, deleting, adding or replacing or changing displayed alphanumeric symbols associated with the selected data feature, providing "boldface", "underline" or "italicized" text in association with the selected data feature, changing the color, shape, or magnitude of the selected data feature, or providing any other alteration in a characteristic of the data feature. Revision of a data feature may involve exporting the object representing the data feature to another application, such as Microsoft PowerPoint® for manipulation or alteration of the feature, after which the data object corresponding to the revised data feature may be returned to programming 14 and/or 18. Actual alteration of the underlying data associated with the data feature may, in some embodiments, not be permitted, or may be subject to user authorization, to prevent patients from altering certain data. Programming 14 and/or 18 may prompt the user to make an edit or revision of the selected data feature. Once the data feature has been revised as desired, the patient data in the selected format may again be displayed in event 120, after which events 130-150 may be repeated. Alternatively, following revision of a selected data feature, event 140 may be repeated to display the revised data feature to the user in an expanded form, after which revision of the selected data feature may again be carried out if desired.

If the user, in event 150, does not elect to revise the selected data feature, event 160 may occur. Event 160 represents completion of a patient data report in a selected data presentation format, with individual data presentation features selectively presented or altered according to the particular needs of the user. As can be seen from the above, the method of the invention allows a user to generate a patient data report that is tailored for particular needs or uses, without having to needlessly re-iterate the generation of complete, but unwanted, patient data reports until a desired final report format is achieved. The completed patient data report, as well as patient data generally, may be stored in the memory of computer 12a-12n for future use, may be transferred to databases 28a-28a-28n via server 16 for use by users within health care LAN 30, may be submitted directly via email attachment or direct transmission of facsimile (fax) to consented relative 38 or physician 40, or as otherwise desired by the patient or patients from whom the report data is derived. Non-patient use of patient data reports may be subject to patient consent, as noted above. In this regard, when individual patients enter data in event 100, programming 14 and/or 18 may present the user, via visual interface, with a request for consent of use of patient data for medical research purposes.

Numerous variations on the above procedure are possible and will be apparent to those skilled in the art. For example, revision of specific data features may be carried out without selective expansion and display of the data features prior to their revision. Following revision of data features in event 190, it may be determined that the selected data presentation format is non-optimal, and event 120 may be carried out again. In some embodiments, display of a selected data presentation format prior to entry of data therein may be omitted, with no display of the patient data report in the selected format being made until after data has been entered or compiled into the report in event 140. In other embodiments, re-entry or re-compilation of data within a report may be desired after revision of specific data features or parameters.

It is again noted that the events described above may be carried out by users of any of computers 12a-12n, 32a-32n, 38 and 40 according to stand-alone programming associated with those computers, or partially or wholly using web-based programming associated with server 34 and or server 16. For example, the user may be a healthcare professional accessing computer 36a-36n, or a relative of a patient that is interested in monitoring the patient's diabetic condition, who would access the patient data server 16 and databases 28a-28n via computer 38.

The methods of the invention are useful for monitoring any patient health condition in which the testing of some analyte associated with a patient-derived sample is completed or carried out numerous times within a certain timeframe. The methods of the invention can be tailored for a specific type of test, type of data collected, a specific health condition, or other consideration. Various other modifications of the inventive methods will suggest themselves to those skilled in the art, and are considered to be within the scope of the invention.

The invention may be utilized with any type of patient data and for creation or generation of any type of patient data report in any report format. In certain embodiments, the invention may be used to provide electronic report generation and patient monitoring for diabetic patients. In such embodiments of the invention, the data presentation formats may comprise a diabetes logbook or data list format. Traditionally, the diabetes logbook has been the focal point of record keeping for the diabetic patient. It is a simple, intuitive record that arranges a patient's daily events into logical groupings, enabling a patient (or the patient's caregivers or relatives) to easily recognize problems, trends, and the level of control needed for a patient afflicted with diabetes.

The logbook, in its simplest form, is a record book that enables a patient to easily record key events (blood glucose readings, medication, exercise, etc). Patient data may be presented in a tabular format, or with patient data arranged according to a daily basis, grouped around meal or other key events. An exemplary data presentation format or report format according to a diabetes logbook is shown in Table 1 (the acronym "BGM" is used to represent "Blood Glucose Measurement").

**TABLE 1.**

| DATE | Breakfast | | Lunch | | Dinner | | Bedtime | | Other | |
|---|---|---|---|---|---|---|---|---|---|---|
| | BGM | Med | BGM | Med | BGM | Med | BGM | Med | BGM | Med |
| Mon. DATE | | | | | | | | | | |
| | Comments: | | | | | | | | | |
| Tues. DATE | | | | | | | | | | |
| | Comments: | | | | | | | | | |
| | (continues for a selected time frame- e.g. MTWTFSS) | | | | | | | | | |

Table 1 represents one way that a diabetes logbook format may be presented to a user via visual interface on a computer display, and includes a series of rows and columns that provide fields for data entry according to blood glucose measurements made before and after meals (breakfast, lunch and dinner), at patient bed time, and for measurements made at other times. Fields are also provided for patient comments. No data is present in the fields, and Table 1 thus represents how a selected data presentation format may be presented to a user by visual display in event 120 described above. The data presentation format of Table 1 provides fields for Monday and Tuesday, but in many embodiments may be extended to include all the days of a week or a longer period of time.

Some exemplary selectable data features for monitoring patients with diabetes in association with the data presentation format of Table 1 include, for example, specific dates, specific data entries on those dates, rows of data, columns of data, blocks of data. A user may, for example, select data associated with post-dinner blood glucose tests by highlighting these data points on the displayed presentation or by selecting the post dinner headings for post dinner blood tests, by appropriately manipulating a cursor via mouse or keyboard. The selectable, expandable features or parameters allow analysis of selected data and produce and display appropriate pie charts, bar graphs or other features associated with the selected data. An example for generating a standard day chart for a diabetes patient may comprise selecting the blood glucose values for a plurality of days displayed in the logbook format of Table 1. Upon selection of the data from the logbook format display, a standard day chart (e.g. glucose mg/dL vs. time of day) may be generated via programming 14 and/or 18. The user may review the standard chart and may want more information on abnormal data points present on the standard day chart. The user may click on the questionable data point so that the details of the data point in question are displayed to the user by, for example, a pop-up window.

The invention enhances user experience in using electronic generated reporting for diabetes care by leveraging the characteristics of a standard diabetes logbook with the "highlight, point and click" aspects of programming 14 and/18 described above. By using a logbook centric view of data, it is possible for a user to easily access other data presentation in a "highlight, point and click" manner.

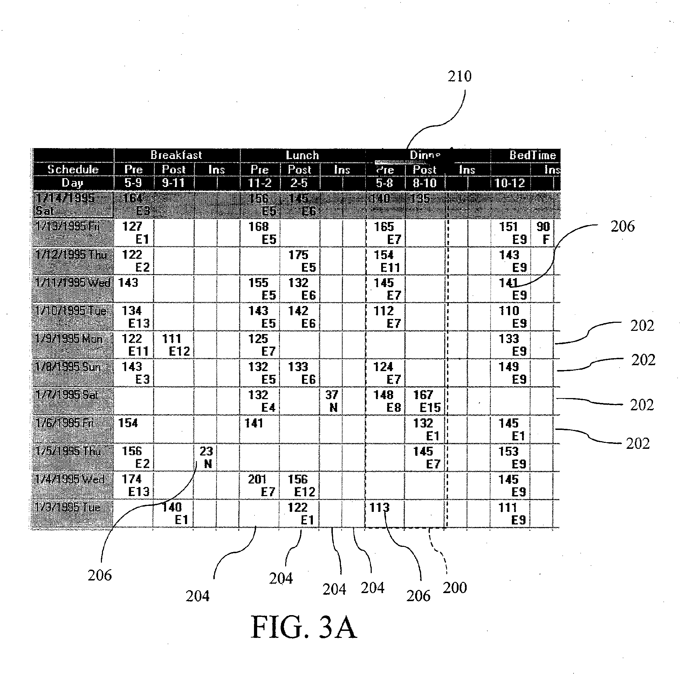

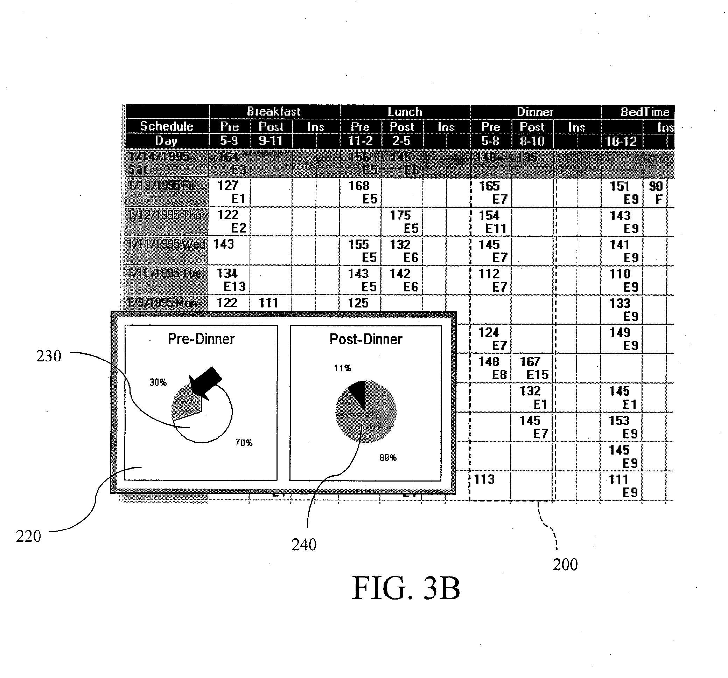

Table 2 presents some specific examples of data presentation or reporting and related descriptions for four different scenarios that may occur in the use of the invention for generating patient data reports on blood glucose levels and monitoring of patient diabetic condition, with reference to the events of FIG. 2. FIG. 3 through FIG. 5, discussed below, represent exemplary visual interfaces that may be associated with the events shown in Table 2, and are also referenced in Table 2.

**TABLE 2**

| Scenario: | Type of Presentation /Reporting | Description/Use Scenario |
|---|---|---|
| 1 | Logbook | The initial report view (FIG. 3A), presented to the user by visual display, and central to all blood glucose data reporting. The logbook presentation format enables the user to view (event 120), enter and edit data (event 150) captured from a blood glucose meter or other device, from manually entered data, or other patient data. |
| | | If the user chooses to select a specific cell or data point (event 130) in the logbook (by, for example, "clicking" on the specific data feature), the details associated with that data point (e.g., date, time, associated events, type of entry, edits, etc.) are displayed (event 140). Such display may involve navigation or hyperlinking to a separate page that specifically displays details associated with the selected data feature, display via a pop-up window, or other display event. |
| 2 | Blood Glucose Level Within Target (pie chart) | The user highlights (event 130) a particular event (breakfast, lunch, dinner, bedtime, etc) for a range of days on the logbook (by selecting, for example, the breakfast BGM column, all breakfast readings in the logbook are included). Such highlighting may comprise, for example, a "click-and-drag" event using a displayed cursor. |
| | | A pie chart report is produced and displayed (event 140, FIG. 3B) showing within and outside of target percentages surrounding that event (e.g. pre-breakfast, post-breakfast). By selecting more than one column (event 130), multiple event "within target" displays (event 140) are produced. |
| | | If the user selects a particular section of a pie chart (event 130), the detailed data that comprises the pie chart may be displayed (event 140) in a pop-up window in tabular form, showing the details. |
| 3 | Standard Day Chart | The user highlights (event 130) a date range (set of rows) in the displayed report format by selecting the range in the date column (FIG. 4A). |
| | | A standard day chart is produced (event 140, FIG. 4B), showing data from the selected days, arranged in a standard day format (by hour or by event as specified b the user). This standard day chart would provide charting of BGM and other data as selected by the user. |
| | | This view is provided by looking at logbook data oriented by rows (days). |
| | | If the user selects a particular data point on the standard day chart, the details of that data will be displayed in a pop-up window. |
| 4 | Trend chart | User highlights (event 130) a data range (set of rows and columns) by selecting the data range in the body of the logbook (FIG. 5A). |
| | | A trend chart is produced (event 140, FIG. 4B), displaying results from the selected data, arranged in a trend chart format (by date/time). This trend chart would provide charting of BGM and other data as selected by the user. The user can interactively turn displayed data (event 150) on or off (e.g., meds, exercise, diet, etc), with this data superimposed on the BGM data. |
| | | This view (event 140) may be provided by looking at logbook data organized as a block of contiguous data, encompassing a date range and a series of events. To look at all data from a range of dates, the user simply highlights the whole block of interest (event 130). If a user selects a particular data point (event 130) on the trend chart, the details of the data will be displayed (event 140) in a pop-up window. |

Exemplary tabular entries for producing a pie chart of target ranges as described in Scenario 2 in Table 2, as well as the corresponding pie chart, are shown in FIG. 3A and FIG. 3B. FIG. 3A is an example of a visual interface display that may be provided for Scenario 2 by the programming of the invention, and includes the data presentation format features of Table 1 discussed above, with rows 202 and columns 204 that define a plurality of cells 206, and with each such cell 206 representing a selectable data feature. FIG. 3A illustrates how a user may select pre and post dinner columns 200 by selecting the header 210 of interest, in this case the Dinner/ Pre and Post columns, which are then highlighted. This selection may be carried out by a conventional "click and drag" operation using a computer "mouse" or other user interface.

Upon selection of pre- and post- dinner columns 200, as shown in FIG. 3B, a pop up window 220 is generated and displayed over the tabular logbook data representation, with a pre-dinner pie chart 230 and a post-dinner pie chart 240 generated or "expanded" and displayed within pop-up display window 220. The pie charts 230, 240 represent target range compliance charts for the patient and show percentages within and outside of target blood glucose ranges for the patient. A consistent, intuitive coloring scheme may be used in association with the visual interface display of FIG. 3A and FIG. 3B. For example, red may be used to indicate "outside target" while green represents "inside target" levels for blood glucose measurements. The window 220 displaying the pie charts may be closed or minimized in a conventional manner via "clicking" on an appropriate icon (not shown). Selecting a specific area of a pie chart 230, 240, may be used to display the data that section in a separate pop up window, showing the details of the data.

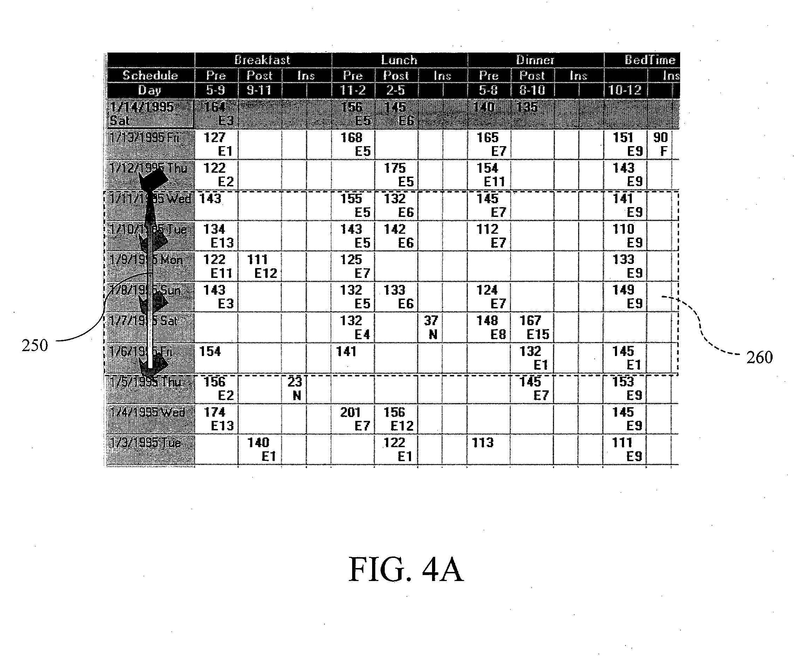

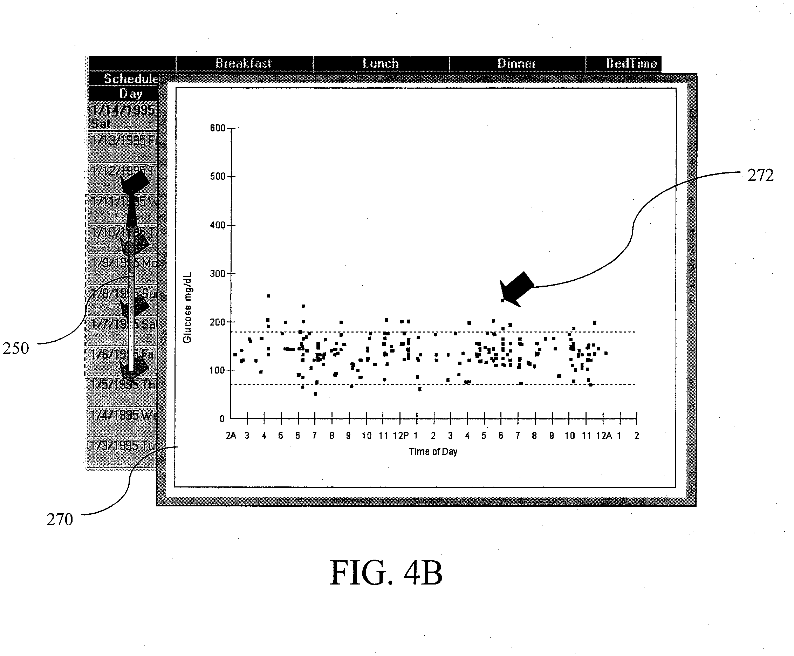

Exemplary tabular format data entries for producing a "standard day" chart for specific days as described in Scenario 3 of Table 2 are shown in the visual interface displays of FIG. 4A and FIG. 4B. FIG. 4A illustrates how a user may select certain days, shown as shaded block 250 in the Schedule Day column, by highlighting the selected block 250. This selection corresponds to the data shown in shaded block 260, and results in generation and display of a window 270 (FIG. 4B) with standard day chart corresponding to the days of the shaded portion 250 of the Schedule Day column. Again, a consistent, intuitive coloring scheme may be used for various data types. The window displaying the standard day chart may be closed or minimized by the user. The user may select a specific data point in the standard day chart in window 270 to provide another pop-up display (not shown) to show the details of the selected data point in pop-up window 270. The user may optionally click on an individual data point as shown by arrow 272 to create yet another display (not shown) that expands upon the selected data point.

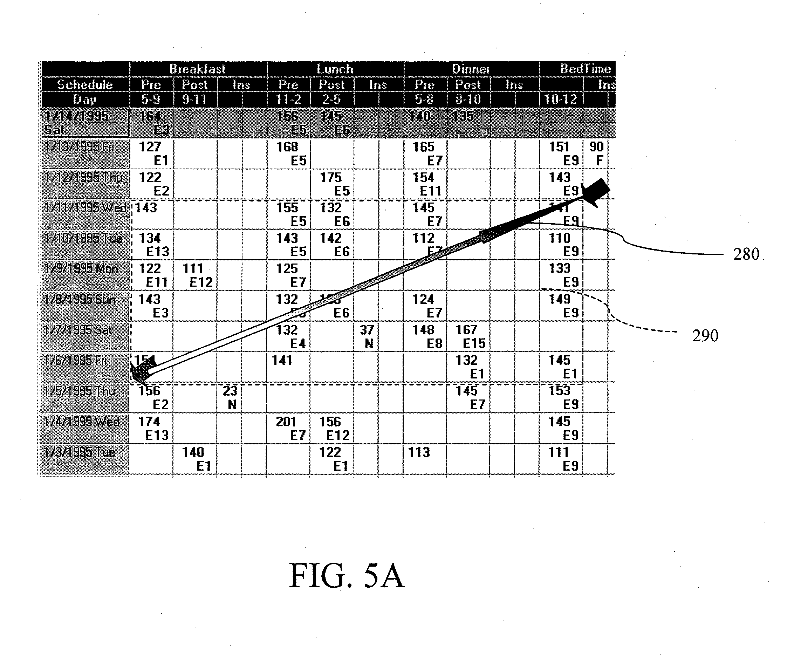

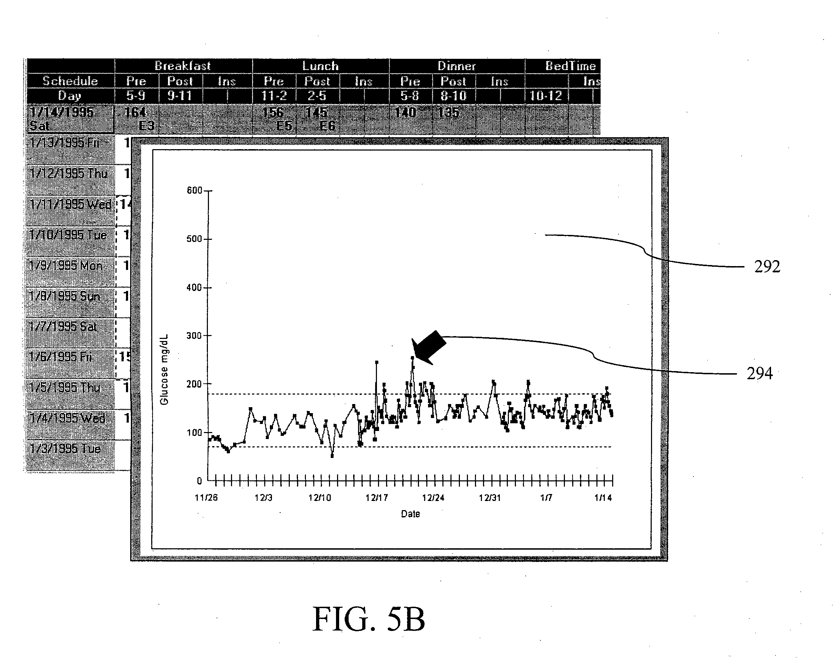

Exemplary tabular entries for producing a "trend" chart for specific days as described in Scenario 4 above are shown in the visual interface displays of FIG. 5A and FIG. 5B. FIG. 5A is illustrates how a user may select certain days and/or events for display of a trend chart via a click and drag operation with a mouse along a diagonal trend line 280 across shaded (highlighted) block 290. Upon selection of the trend line 280, a pop up window 292 (FIG. 5B) showing the corresponding trend chart is generated and displayed. The user may select a data feature associated with display 292, such as the data point shown by arrow 294, to create yet another display (not shown) that expands upon the selected data point.

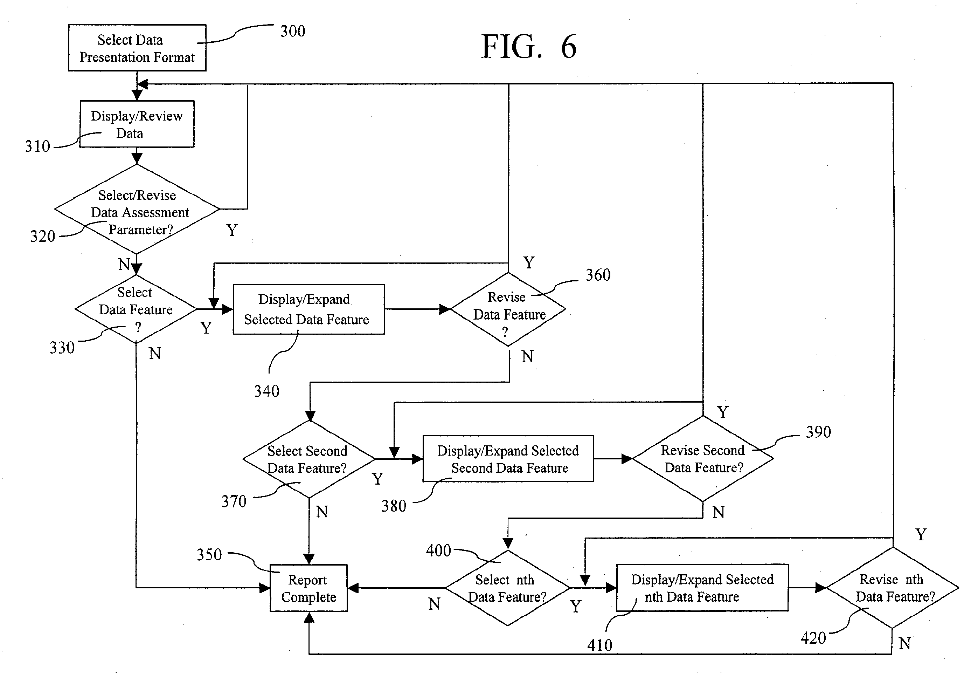

Referring now to FIG. 6, another embodiment of the methods of the invention is shown. The events of FIG. 6 may be carried out using one or more of the computers and programming shown in FIG. 1 and described above. Once again, it should be understood that the arrangement or order of the events is only exemplary and may be varied as required for particular uses of the invention.

At event 300, the programming of the invention to provide for selection of a data report format for presentation of patient data. The report format may be in the form of a logbook or data list of the type commonly used for diabetes patients as noted above, or in other format. User selection of a particular data presentation format may be prompted by a suitable visual interface presented by the programming via visual display. The visual interface may utilize "pull-down" menus, "help" menus, graphical user interface (GUI) icons upon which a user may "click" with a mouse to make a selection, text fields in which a user may enter alphanumeric character strings using a keyboard, or other conventional visual interface tools as described above.

At event 310, patient data is displayed for the user, via programming-generated visual interface, in the selected presentation format.

At event 320, the user may select and revise or edit a data assessment parameter associated with the displayed data presentation format. A data assessment parameter is any parameter that affects the type or properties of data that is displayed in the selected format. A data assessment parameter may comprise, for example, a selectable patient name or patient identifier, a selectable date range for patient data, selectable threshold values for data to be displayed, or other parameter. The programming of the invention may provide for user selection of data assessment parameters by presenting "clickable" icons, text fields, or other interactive display features that allow a user to make a selection using a mouse, touch screen interface, or other interface device.

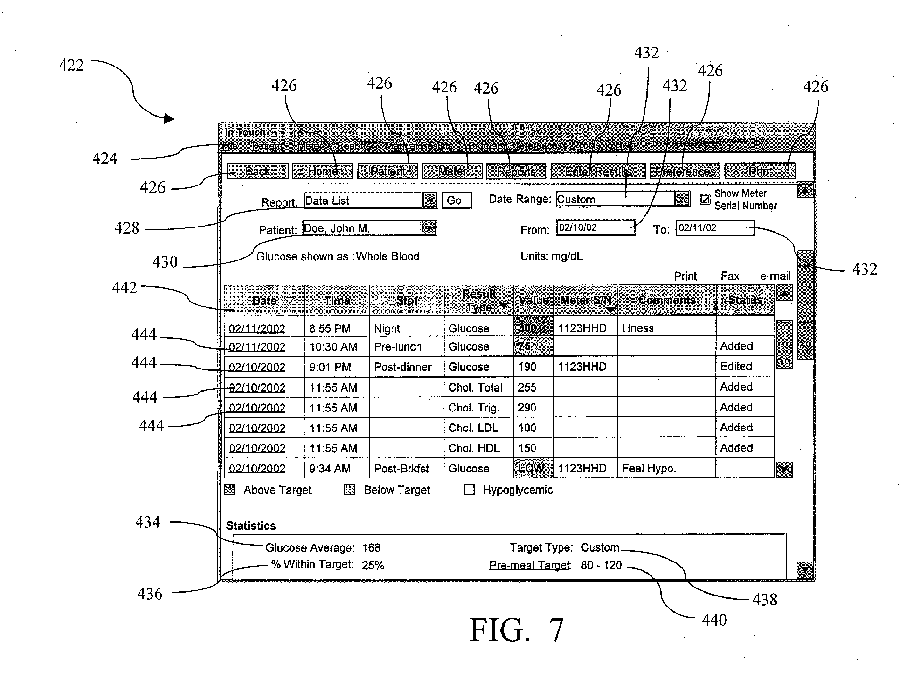

If the user elects to revise or edit a data assessment parameter, event 310 is repeated, with the displayed data having been refreshed or revised according to the changed data assessment parameter. For example, if the user elects to change the patient name for the displayed data, data associated with the selected patient will be displayed for the user. If a date range has been changed, the patient data associated with the revised dates is displayed. If a data threshold value is changed, the patient data that exceeds (or falls below) the threshold value is displayed. Selectable data assessment parameters are discussed further below with reference to FIG. 7. If the user does not choose to alter any data assessment parameters, event 330 may occur.

At event 330 the user may select a particular data feature that is displayed in event 310. A selectable data feature may comprise, for example, a particular row(s) or column(s) of a tabular data presentation format, a particular cell(s) within a row or column, a selected "wedge" of a pie chart, a selected region of a graphical data representation, data associated with measurements taken on a particular day or at a particular time of day, measurements associated with resting or stimulated conditions, measurements associated with meals, or any feature, component or aspect of the patient data as described above. Data features may be presented to the user as GUI devices, "clickable" icons, manipulable first class objects, or other interactive visual interface element. Selection of data features may occur by "clicking" on the displayed data feature of interest or an icon representing that data feature using a "mouse". If the user selects a data feature in event 330, event 340 follows. If the user does not wish to select any data features associated with the patient data presented in the selected format, generation of the patient data report is complete, and event 350 may occur.

At event 340, the data feature selected in event 330 is displayed via visual interface. The display of the selected data feature may comprise a visually expanded version of the data feature. The expanded data feature may be presented to the user in a separate window or frame such as a "pop-up" window, which may overlay or be superimposed on the display of the data presentation format, on a separate display page, or by other form of display. Data feature selection may involve navigation between hyperlinked pages such as HTML pages, such that the overall data report format is associated with a first display page, and selectable data features are associated with other display pages that are accessed in a "drill-down" manner as described above. The expansion and display of individual data features in event 340 provides the user with the opportunity to analyze specific data and, if desired, change specific features of the data presentation format to optimize the final data report.

At event 360, the user may elect to revise or otherwise change the selected data feature displayed in event 340. Revision of the data feature in event 360 may comprise deleting, adding or replacing or changing displayed alphanumeric symbols associated with the selected data feature, providing "highlighting", "boldface", "underline" or "italicized" text in association with the selected data feature, changing the color, shape, or magnitude of the selected data feature, or providing any other alteration in a characteristic of the data feature as described above. Revision of a data feature may involve exporting the object representing the data feature to another application for manipulation or alteration of the feature.

Once the data feature has been revised as desired in event 350, event 340 may be repeated to display the revised data feature, after which the user may again elect to make a revision and repeat event 360. Alternatively, event 310 may be repeated such that the patient data report is again be displayed in the selected format with the revised data feature, after which events 320-340 may be repeated.

The expanded data feature displayed in event 340 may include additional data points or features that are associated with the data feature selected in event 330, and which were not displayed until event 340. For example, if the data feature selected in event 330 represents a particular date, the expanded display of that date in event 340 may include multiple patient data measurements for the particular date. The user may wish to selectively edit one or more of these additional or secondary data features. In this regard, if a user at event 360 does not wish to revise the expanded data feature selected in event 330 and displayed in event 340, but instead wishes to select and revise one of the additional data features that arises in the display of event 340, event 370 may be carried out. If the user does not wish to select and revise one of the additional data features, event 350 may occur.

In event 370, the user may select a second data feature associated with the data feature display of event 340. Selection of the second data features may occur by "clicking" on the displayed data feature of interest as described above. Following selection of the second data feature, event 380 occurs, and the selected second data feature is displayed for the user. This display may be in the form of a pop-up window, a separate display page that is hyperlinked to a page displayed in event 340, or other form of display as noted above. The user may wish to revise the data feature displayed in event 380, after which event 380 may be repeated to display the revision for the user. Alternatively, event 310 may be repeated at this point to provide the user with a view of the entire report.

Once again, the data feature selected in event 370 and displayed in event 380 may give rise to additional data features not previously displayed to the user. Thus, if at event 390 the user does not wish to revise the particular data feature selected at event 370, but wishes instead to select and edit one of the newly displayed data features, event 400 may occur.

At event 400, the user may select an nth data feature associated with the data feature display of event 380. Selection of the nth data features may occur by "clicking" on the displayed data feature of interest as described above, after which event 410 occurs and the selected nth data feature is displayed for the user. The user may revise the data feature displayed in event 410, after which event 410 may be repeated to display the revision for the user, or event 310 may be repeated to provide the user with a view of the entire report. Additional iterations of events 400-420 may occur as required until the user has had an opportunity to revise any desired data feature associated with the patient data report. If the user does not wish to select an nth data feature at event 400 or, having made such a selection but not wishing to edit or revise the selection at event 420, event 350 may occur.

If the user, in event 420, does not elect to revise the selected data feature, event 350 may occur. Event 350 represents completion of a patient data report in a selected data presentation format, with individual data presentation features selectively presented or altered according to the particular needs of the user. As can be seen from the above, the method of the invention allows a user to generate a patient data report that is tailored for particular needs or uses, without having to needlessly re-iterate the generation of complete, but unwanted, patient data reports until a desired final report format is achieved. The completed patient data report may be stored in the memory of computer 12a-12n for future use, may be transferred to databases 28a-28a-28n via server 16 for use by users within health care LAN 30, may be submitted directly via email attachment to consented relative 38 or physician 40, or as otherwise desired by the patient or patients from whom the report data is derived. Non-patient use of patient data reports may be subject to patient consent, as noted above. In this regard, when individual patients enter data in event 100, programming 14 and/or 18 may present the user, via visual interface, with a request for consent of use of patient data for medical research purposes.

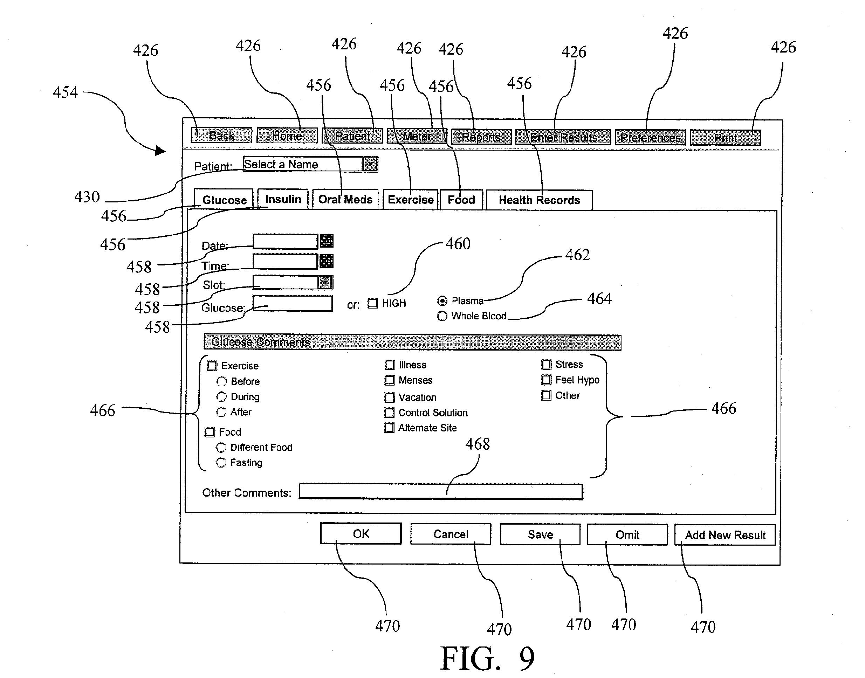

A specific example of the events of FIG. 6 is provided in the visual display illustrations of FIG. 7 through FIG. 9. FIG. 9 illustrates an exemplary display page 422 of a patient data report that may be created and displayed by the programming of the invention that allows interactive patient data report generation in accordance with the invention. The page 422 is shown in a data list format for patient blood glucose data. The programming of the invention provides a set 424 of pull down menus for various software tools, shown in FIG. 7 as "Patient", "Meter", "Reports" and "Manual Results, as well as conventional tools such as "File", "Program Preferences", "Tools" and "Help".

Display page 422 also presents several icons 426 that represent various operations provided by the programming of the invention. In the embodiment of FIG. 7, icons 426 are clickable icons that a user may select to navigate directly to specific display pages. The pages may be in the form of HTML pages, XML pages, or other arrangement that allows hyperlinking between pages in a conventional manner. Icons 426 thus include "Back", "Home", "Patient", "Meter", "Reports", "Enter Results", "Preferences" and "Print" to provide navigation to commonly used pages that are hyperlinked to page 422.

Display page 422 provides a text field 428 that allows a user to select a specific patient data report format in accordance with the invention. A "Data list" format is shown in text field 428, and the displayed page 422 is presented to the user in a data list format. A user may use a pull-down menu associated with field 428 to select other patient data report formats, or may manually enter the desired report format via keyboard. Numerous other formats for presenting patient data in a report may be used as described above.

The invention provides for user selection of data assessment parameters as described above with reference to FIG. 6. Text field 430 provides a selectable "patient" parameter, and a user may use a pull down menu associated with field 430 to select a patient name, or manually enter a patient name, to display data associated with the selected patient in a data list format on display page 422. Text fields 432 provide a selectable data range parameter, and allow a user to use a pull down menu to select a date range such as a day, week, month, etc., or to enter a start date and end date for a date range. Upon selecting a particular date range, the corresponding patient data for that date range is displayed on page 422.

In the embodiment shown in FIG. 7, selectable data assessment parameters associated with patient blood glucose levels are also provided. Field 434 allows a user to enter a "Glucose Average" numerical value, and field 436 allows user entry of a "% within target" numerical value. Field 438 allows user entry of a particular type of target, and field 440 allows a user to select numerical ranges for a particular target such as a "Pre-meal target" blood glucose level. A user may select and revise these parameters as desired to selectively display patient data as "Above Target", "Below Target", or "Hypoglycemic" according to selected threshold values or ranges. Blood glucose measurements that are outside of target values or ranges may be shown as highlighted using colors in an intuitive manner.

Display page 422 includes a block 442 of data features 444 arranged in rows and columns according to the data list presentation format. The data features, some or all of which may be selectable for expanding and editing, include individual dates, times, and time slots associated with patient data measurements, result types, numerical values for results, sample meter serial numbers, patient comments, and result status. Blood glucose level data features in the "Value" column are shown as highlighted or shaded as "Above Target" or Below Target" according to the target parameters selected in fields 434, 436, 438, 440. The sort order of the displayed data features 444 may be revised by clicking upon column headers such as "Date", such that data features may be shown from most recent to least recent, or vice versa.

A user may select a data feature by "clicking" on the selected data feature presented on display page 422 to create a display of the selected data feature in expanded form. Display of the selected data feature may comprise a pop-up window displaying the data feature, result in navigation to another, hyperlinked display page specific for the selected data feature, or other form of visual display for the selected data feature. In the embodiment shown in FIG. 7, underlined data features represent data features that may be selected for specific display and editing.

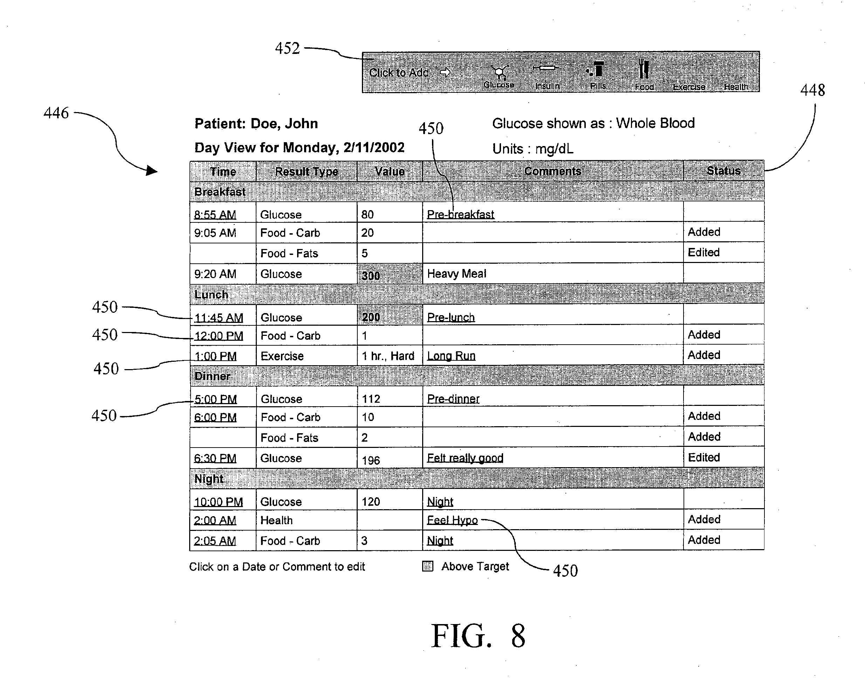

FIG. 8 shows a display page 446 resulting from a user "clicking on the data feature 444 in FIG. 7 corresponding to the date "02/11/2002". Display page 446 presents the user with data from an individual day, corresponding to the selected date, for the data list report shown in FIG. 7. Display page 446 in this embodiment represents a page that is hyperlinked to display page 422 of FIG. 7, to which the user can navigate by clicking on the "02/11/02" data feature on page 422. Display page 446 is thus accessed in a "drill-down" manner from display page 422.

Display page 446 includes a block 448 of data features 450, some or all of which may be selectable for expanding and editing. The data features 450 include individual times, result types, numerical values for results, patient comments, and status, which are arranged in rows and columns according to the data list format. Blood glucose level data features in the "Value" column are shown as highlighted or shaded as "Above Target" according to the target parameters selected in fields 434, 436, 438, 440 on page 422 as described above. Display page 446 also includes selectable icons 452 that allow a user to edit the "comments" data features" by clicking upon the appropriate icon. These icons allow the user to enter a brand new record/result of the type signified by the icon. Entry of such new data may be carried out manually by the user.

Clicking on a specific data feature 450 such as a time or comment navigates the user to manual user entry screen display, shown in display page 454 in FIG. 9. Display page 454 represents a manual data entry form for patient users. Display page 454 includes a plurality of "Tab" menus 456 for "Glucose", "Insulin" "Oral Meds", "Exercise", "Food" and "Health Records" that allow a user to navigate to displays specific for those topics. The "Glucose" tab is illustrated in FIG. 9, and includes fields 458 for user entry of date, time, time slots, and glucose level. Included with fields 458 are icon 460 that a user may select to indicate a "High" glucose reading, and icons 462, 464 that allow a user to indicate whether the glucose measurement was obtained from plasma or whole blood.

Glucose tab 456 includes icons 466 that allow a user to select specific comments associated with a glucose measurement, such as "Exercise (before/during or after measurement), "Food", "Illness", "Menses", "Vacation", "Control Solution", "Alternate Site", "Stress", "Feel Hypo" (hypoglycemic), and "other". A text field 468 is provided to allow the user to enter other comments. Display page 454 is shown with clickable icons 426 navigating the user directly to specific display pages as described above. Page 454 also includes conventional button icons 470 for saving results, canceling an operation, adding a new result, etc.

The specific visual displays provided in the drawings and described above represent only a few of many possible display arrangements that may be used with the invention, and should not be considered as limiting. Numerous other visual display arrangements for interactive patient data reports will suggest themselves to those skilled in the art upon review of this disclosure, and such display arrangements are also considered to be within the scope of the invention. The drawings and description provided herein show exemplary embodiments of the invention for use with patient glucose data. The invention, however, may be used with any type of patient or medical data.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. A method for creating a patient data report, comprising:
(a) selecting a report presentation format;
(b) displaying patient data in said report presentation format;
(c) selecting a data feature associated with said patient data; and
(d) displaying said selected data feature.

2. The method of claim 1, further comprising revising said selected data feature.

3. The method of claims 1 or 2, further comprising generating said patient data.

4. The method of claims 1, 2 or 3, further comprising compiling said patient data in said selected report presentation format.

5. The method of any of the preceding claims, wherein said displaying said patient data in said report presentation format comprises generating a first visual display page, and wherein said displaying said selected data feature comprises displaying a second visual display page hyperlinked to said first visual display page.

6. The method of any of the preceding claims, wherein said displaying said selected data feature comprises displaying said selected data feature in a window overlaying said patient data report presentation format.

7. The method according to any of the preceding claims, wherein said displaying said selected date feature comprises a drill down operation.

8. The method according to any of the preceding claims, wherein said displaying said selected data features comprises displaying said selected data feature in a window overlaying said-patient data report presentation format.

9. The method according to Claim 8, wherein said patient data presentation format comprises a tabular logbook format.

10. A system for generating patient data reports, comprising programming for performing the method according to any of Claims 1 to 9.
